# EUROPEAN PATENT SPECIFICATION

(11) **EP 2 174 559 B1**
(45) Date of publication and mention of the grant of the patent: **23.07.2014**
(21) Application number: 08761512.6
(22) Date of filing: 15.04.2008
(51) Int. Cl.: A23L 1/275, C09B 61/00, A23K 1/16, C07C 49/603, C07C 403/24

(54) **WATER-DISPERSABLE CAROTENOID FORMULATION**
WASSERDISPERGIERBARE CAROTINOIDZUSAMMENSETZUNG
FORMULATION DE CAROTÉNOÏDES POUVANT ÊTRE DISPERSÉE DANS L'EAU

(30) Priority: 28.07.2007 ES 200702142
(43) Date of publication of application: 14.04.2010
(73) Proprietor: Investigaciones Químicas y Farmacéuticas, S.A., 43080 Tarragona (ES)
(72) Inventor: FERRATER MARTORELL, Joan Carles, E-43080 Tarragona (ES); FERNÁNDEZ MARTÍN, Juan Antonio, E-43080 Tarragona (ES); RIBERA RUIZ, David, E-43080 Tarragona (ES); VISO ACOSTA, Antonio, E-43080 Tarragona (ES)
(74) Representative: Brewster, Andrea Ruth
(86) International application number: PCT/ES2008/000266
(87) International publication number: WO 2009/022034

(56) References cited:
- WO-A1-91/06292
- ES-A1- 2 265 787
- ES-T3- 2 101 512
- US-A- 3 998 753
- US-A- 5 997 922
- "Opinion on the scientific commitee on animal nutrition on th euse of canthaxanthin in feedingstuffs for salmon and trout, laying hens, and other poultry", EUROPEAN COMMINSION, HEALTH & CONSUMER PROTECTION DIRECTORATE GENERAL, 17 April 2002 (2002-04-17), XP002599132,
- BALNAVE D. ET AL.: 'Relative efficiencies of yellow carotenoids for egg yolk pigmentation' ASIAN-AUSTRALIAN JOURNAL OF ANIMAL SCIENCE vol. 9, no. 5, 1996, pages 515 - 517, XP009137615

## Description

This report describes a water-dispersable carotenoid formulation in form of a liquid which contains a mixture of canthaxanthine and marigold soap, which given the preparation characteristics thereof, has a pigmenting potential on eggs and animal tissues much greater than that obtained when these xanthophylls are applied separately.

Canthaxanthine, as well as Lutein and Zeazathin, the latter two basic components of marigold soap, belong to a family of products named carotenoids characterized by containing a high number of conjugated double bonds, which is the basis of many of its applications. The name xanthophylls appears often in the bibliography which refers to carotenoids containing oxygenated functions in their structure, mainly keto or hydroxyl groups.

In fact, the extended conjugation of the double bonds that make up its carbohydrate structure gives the carotenoids a generally intense colour. This quality is highly valued, especially in the food industry.

These products have a particular application as colouring additives in the animal feed industry. They are used significantly in the pigmentation of egg yolk and chicken flesh, as well as in the tissues of other animals such as salmonids and crustaceans.

Its use as a protector against light-induced oxidation is also described, which can prevent many illnesses, including cancer. References to chemical prevention of pre-cancerous states, or prophylaxis of cardiovascular disease, frequently appear in the bibliography.

One of the most significant limitations associated with the manipulation and use of carotenoids lies in its relatively low stability.

There is a lot of literature about the multitude of degradation routes through which carotenoids may develop. They also usually degrade in contact with air, generating fragmentation or oxidation products such as epoxides. In fact, this behaviour is the basis of its function as an antioxidant agent, as its own oxidation avoids the development of degradation of the rest of the components.

Equally, the presence of acids, more or less strong, causes degrading reactions with the abundant formation of fragmentation products. These processes are favoured by the presence of light, as well as by radical generating compounds, among others.

Obviously, the behaviour against some of these agents is a consequence of its molecule in a large number of conjugated double bonds that, as has been pointed out, is the reason for its antioxidizing activity.

One of the important aspects therefore which will ensure its industrial application as a pigmenting agent, especially in food, will be based on guaranteeing a high stability against all the agents mentioned above.

The invention described in this report is with regard to a formulation that contains a mixture of characteristics made with a synthetic xanthophyll, canthaxanthine, a saponified vegetable extract, marigold soap, appropriate for use as a pigmenting agent in the animal feed industry. This formulation, as described below, is a significant advance with regard to colouring efficiency compared especially with the separate application of these two products.

The surprising increase in efficiency in the formulation developed is substantiated by numerous aspects concerning greater deposit on animal tissue, an increase in carotenoid stability due to additional protection against degrading processes and its more uniform and homogenous distribution in the food that optimizes the pigmenting power of the xanthophylls involved.

### STATE OF THE ART.-

The bibliography has many examples of xanthophyll use as animal food colouring additives for the pigmentation of eggs and tissue.

Marigold extract is one of the most important natural sources because of its high xanthophyll content, fundamentally Lutein and Zeaxanthin that, as they are in ester form, are subjected to a process of saponification prior to their use as an additive.

One of the first preparation experiments of free xanthophylls from marigold is that described and claimed in the year 1970, in US 3523138. In it, the marigold extract is subjected to saponification with KOH or NaOH in alcohol. The partial neutralization with phosphoric acid allows the preparation of a concentrate with high purity in total xanthophylls and is appropriate for use in animal feed.

In the American patent US3535426 belonging to Eastman Kodak, a stabilization of xanthophylls from natural vegetable extracts process is described which consists of the saponification of marigold flour using high quantities of potassium hydroxide, ethanol and water. The saponification process was carried out in a nitrogen setting and lasted two hours. Once the saponification was neutralized, a saturated fat was added to it, and finally it was dried to obtain a powder. A similar process is that described in patent US3997679 belonging to CPC International.

There are many other examples that describe the saponification of marigold oleoresin in different conditions with the aim of preparing a soap that, appropriately excipiented is used as is in the preparation of a premixture for application directly on animal feed. Of special interest is the general process claimed by IQF in 1995 in ES2099683. According to this method, high purity concentrates are obtained through the saponification of fatty extracts of marigold of paprika by the action of an alcoholic dissolution of NaOH or KOH, optionally with the presence of propylene glycol.

The bibliography also has many examples of the preparation of xanthophylls from marigold soap with a high level of purity. An example which illustrates this aspect is that described by Tyczkowski and Hamilton in Poultry Sci., 70 (3), 651-4 (1991). Following their procedure, high purity concentrates of lutein are obtained. The method consists of the saponification of the marigold extract through the action of alcoholic KOH and its extraction with a mixture of solvents (HEAT). According to the authors, after the organic phase separation and its partial evaporation crystal lutein is obtained, whose recrystallization in hexane-acetone allows the isolation of crystals with a lutein purity of more than 99%.

Many examples of the use of synthetic xanthophyll, Canthaxanthine have also been described as a colouring additive for the pigmentation of animal tissue, which gives it an attractive red tone.

In recent years, different procedures for the formulation of carotenoids have been developed which try to guarantee, in addition to its stability, above everything, its correct absorption and therefore its bioavailability. These characteristics, as a whole, are those that determine the type of formulation to be used.

Many methods to improve pigmenting power and increase absorption have been described, for which it has been attempted to reduce the size of the crystals to fewer than 10 microns. In Chimia, 21, 329, (1967) formulation in colloid oil is described, which on the other hand has a stabilizing effect, as shown in Food Technol., 12, 527, (1958). Its use in such a way shows significant limitations because of its low miscibility with water.

Moreover, its aqueous preparation of carotenoids and in particular canthaxanthine, is much more difficult. In fact, it must be taken into account that the aqueous solubility of carotenoids, given its relatively apolar structure, is very low, which means a significant limitation in obtaining a high level of pigmentation.

Various plans have been tested, for example, the use of water-miscible organic solvents, such as ethanol or acetone, and in contact with water which would dissolve the carotenoid and which would generate very small absorbable particles. Such are the examples described in EP65193 and in US4726955. However, the solubility of the carotenoids, even in these types of solvents, is very low and would need large quantities of solvent to achieve acceptable results.

Other processes involve formulations using appropriate excipients. The carotenoid is thus dissolved in oil, according to the method described in DE 642307, or in chloroform, in accordance with DE 861637 and is sprayed on an excipient with starch, powdered milk or similar. However, these formulations in addition to not dispersing easily in water, show significant problems in stability against oxidation.

To this end, the best results were obtained with the incorporation of gelatine in the preparation of the emulsions, as described in Chimia, 21, 329, (1967) and in FrP1056114 and US2650895.

The greatest advance regarding water-dispersible formulations consists of the examples described in Chimia, 21, 329, (1967), DE 1211911 and DE2534091. According to these examples, the carotenoid dissolves in a solvent immiscible in water, of a chloroform or dichloromethane type, and the solution obtained is emulsified by homogenization with an aqueous solution of gelatine and sugar. The elimination of the organic solvent creates a gel that, as an option, can be sprayed over a desiccant excipient with which a truly water-dispersible formulation is obtained. The stability of the xanthophylls thus increases considerably and at the same time ensures its bioavailability up to a certain point.

The bibliography has other examples of formulations of canthaxanthine and other similar carotenoids such as liquid dispersions in the presence of pH controlled specific emulsifiers, or formulation techniques following microencapsulation processes, i.e. making small capsules containing the pigment inside, protected from the direct action of the oxygen in the air.

In the particular case of carotenoids from vegetable sources, such as Lutein or Zeaxanthin, whose most important source is the Marigold flower, the miscibility in the aqueous means is achieved through the saponification of the fatty acid esters and hydroxyl groups of these xanthophylls, which causes the formation of watersoluble soaps and simultaneously the liberation of carotenoids.

References to the use of marigold soaps, mainly consisting of Lutein and Zeaxanthin, are common in the bibliography, such as those previously stated, as is or supported on a mineral excipient for the creation of premixtures in animal food. This can be considered its most common formulation.

ES 2265787 describes an aqueous emulsion containing saponified marigold oleoresin which can be used as a colorant for broiler chicken and egg yolk.

"Opinion of the Scientific Committee on Animal Nutrition on the use of canthaxanthin in feedingstuffs for salmon and trout, laying hens, and other poultry", European Commission, Health & Consumer Protection Directorate General, 17 April 2002, describes the use of canthaxanthine as a red colourant in feed for salmon and trout, laying hens and other poultry.

Therefore, as has been shown, the bibliography has a wealth of information about synthetic carotenoid formulations in the form of a water-dispersible and plant xanthophyll product in dispersion in the soaps created "in situ" in the saponification process of its esters.

The commercial availability of these products has led to their separate use in the preparation of the feed. The canthaxanthine and marigold xanthophylls formulations are mixed in the form of solids and in the appropriate proportion to achieve the desired colour. This is common practice.

However, there is no precedent of use for the preparation of pigments for animal feed in a water-dispersible formulation that simultaneously contains the synthetic carotenoid, the Canthaxanthine, and the plant xanthophylls from the saponified marigold extract; see for instance D. BALNAVE et al. AJAS 9/5, 515-517 (1996).

### THE INVENTION.-

A liquid carotenoid formulation, which contains a mixture of Canthaxanthine and marigold soap, is described in this report, suitable for use as a pigmenting additive in the preparation of premixtures and animal feed and with significant advantages compared to those described and marketed so far.

According to a first aspect of the invention, there is provided a water-dispersible formulation of carotenoids in liquid form, comprising a mixture of Canthaxanthine and saponified marigold oleoresin, which contains between 1 and 500 parts of total carotenoids for each 1000 parts of solution suitable for use as a pigmenting agent in the animal feed industry.

As can be checked below in the detailed description of the invention, this formulation therefore shows specific characteristics that allow a significant increase in pigmenting activity to be obtained in respect to that obtained by applying the carotenoids separately.

Comparative studies have been conducted on the pigmentation achieved in egg yolk used in feed that contains the formulation, which is the subject of this invention, and other feed that contains the two commercial products in powder form (Canthaxanthine and marigold xanthophylls).

In these tests, for the control of the evolution of colouring obtained throughout the treatment's duration period, values reached has been recorded on the "a" and "b" Minolta scales.

The results obtained are conclusive and show that, on average, around 20% less total carotenoids are needed using the liquid formulation which is the subject of this invention, in order to reach the same level of pigmentation.

Therefore, the liquid formulation described in this report shows a significant improvement in pigmenting efficiency in eggs with regard to that achieved using separate powder products. This surprising behaviour can be found as a sum of various factors.

From a microscopic point of view, a significant improvement must be highlighted, as the addition of soap favours the homogenous dispersion of the carotenoid particles in the feed. This allows considerable improvement in the carotenoid dosing and thereby regulated the dose and daily intake. The immediate consequence is an improvement in the homogeneity of the pigmentation results in the tissues on which it is deposited.

From a metabolic point of view, a significant advantage is connected to the already known and fully explored carotenoid absorption difficulty at the intestinal level. In accordance with the formulation raised here, the liberation of carotenoids in the food is immediate, which will favour its absorption and therefore increase its efficiency.

At a practical level, an additional significant aspect must be highlighted. The preparation of a liquid product consisting of a matrix with a high proportion of soap will thereby allow its application directly on the food in the final phase of its preparation. An efficient and homogenous covering of the particles that make up the food is thus given.

One characteristic of the formulation described here, and which constitutes a significant argument for the increase in pigmenting efficiency, is the increase in the carotenoids' stability against oxidation and other connected degradative processes.

To confirm this improvement, numerous comparative studies have been conducted on the stability of this type of mixture against commercial products that do not contain added soap. Some results are shown in Table I.

The study of accelerated stability was performed raising the temperature to 70°C in the presence of air. The retention percentages are expressed as a proportion of canthaxanthine remaining following the thermal treatment.

**Table I.- Comparative study of the accelerated stability of liquid pigments.-**

| **Time (h)** | **Canthaxanthine 1%** | | **CTX+JBM (*)** | |
|---|---|---|---|---|
| | HPLC | retention % | HPLC | retention % |
| 0 | 95.66 | 100% | 16.67 | 100% |
| 17 | ---- | 94.3% | ---- | 98.52% |
| 48 | 93.25 | 84% | 16.16 | 93% |

| | | | | |
|---|---|---|---|---|
| (*) CTX+JBM is a preparation which is the subject of this invention and which consists of a mixture of canthaxanthine formulate with marigold soap, prepared in accordance with that described in this report. | | | | |

A series of very significant conclusions can be taken from the above table. First of all, it shows that the thermal treatment at 70°C and in the presence of air causes a significant degradation in the canthaxanthine formulate obtained by the usual methods. After 17 hours, the retention is 94.3% and after 48 hours in these conditions it goes down to 84%.

With regard to the product prepared in accordance with this CTX+JBM report, the thermal treatment causes a much lesser degradation, the retention percentage being 98.52% after 17 hours, and remains at 93% after 48 hours.

The results obtained show that although the gel, which the canthaxanthine is certainly stable in its usual formulation, the addition of soap made from the saponification of marigold oleoresin causes an extra stabilizing effect, even in extreme degradative conditions.

It has been demonstrated that this effect does occur, even with relatively small additions of this soap, which allows measuring of the additional quantity of soap depending on necessities.

Although the reason for this increase in stability has not been able to be completely reasoned for now, a series of arguments which may help to clarify this fact can be noted.

The soap created from the saponification of the marigold oleoresin contains a high proportion of natural products, rubbers, waxes, etc., which may serve as protective agents, lowering the possibility of contact with atmospheric oxygen. This protective effect would be similar to that which these same products have on the xanthophylls contained in the marigold oleoresin, which in those conditions show high stability.

Moreover, the soap contains a high percentage of xanthophylls, epoxides and other carotenoid derivatives that, because of their antioxidant power, would also serve as protective agents against the degradation of the canthaxanthine itself.

Finally, the surface-active character owing mainly to the fatty acids' alkaline salts coming from the oleoresin saponification may help to decrease the surface contact of the canthaxanthine crystals with air and thereby protect it against oxidation and contribute to its stability.

Moreover, it has been confirmed that the addition of soap does not affect the bioavailability of the canthaxanthine in the final product, which, through the mere fact of ensuring carotenoid stability, an increase in its pigmenting activity is expected.

It is important to point out another aspect which although does not mean an improvement from the point of view of stabilization or pigmenting power, it does from the point of view of its commercial use. Thus, the addition of a red colorant, such as canthaxanthine, from a mixture of yellow xanthophylls, such as those contained in the marigold soap, allows them to be combined and thereby give a wide range of attractive colours in the preparation of additives for the food.

From the industrial point of view, it is important to point out the simple assembly necessary for the preparation of the formulate. Compared with the more or less complex industrial assembly described for other types of formulations shown in the bibliography, in the case described here, the use of a reactor equipped only with an effective agitator and temperature control is sufficient.

Another important advantage of the procedure described here lies in the reduction of costs that has been achieved in the overall process about that already described. Thus, apart from the already stated simplicity in assembly, the nature of the reagents and the reaction conditions and isolation method of the final products contribute to lowering manufacturing costs, making this procedure a good alternative from an industrial point of view.

### DETAILED DESCRIPTION.-

As has been stated above, a water-dispersible carotenoid formulation in liquid form is described in this report which contains a mixture of canthaxanthine and saponified marigold oleoresin and which given their preparation characteristics demonstrate a much higher pigmenting power on eggs and tissue than that obtained by the usual method which consists of the application of these xanthophylls separately.

From the start, the canthaxanthine is generally prepared by synthesis, although to carry out its preparation, albeit uncommon, the xanthophyll-enriched product, obtained by extraction from natural sources may be used.

The first phase of the process for the preparation of the formulation which is the subject of this invention involves obtaining a hydrosoluble form from the canthaxanthine.

There are different procedures for the formulation of the canthaxanthine for the preparation of colouring additives suitable for food. The preparation of a water-dispersible gel is normal by dissolving the canthaxanthine in an organic solvent immiscible with water and put in contact with an aqueous solution that contains carbohydrates and proteins together with auxiliary agents. The elimination of the solvent allows for the preparation of a gel that is dispersible in an aqueous medium.

The formulation process of this gel can be completed by spraying it on a bed of desiccant agent with which the preparation of beadlets is feasible. Both the precursor gel and the sprayed product can be used as precursors of the formulation described here.

A second phase consists of the preparation of a hydrosoluble form that contains the xanthophylls Lutein and Zeaxanthin, which is taken from a plant source rich in these xanthophylls, namely marigold oleoresin.

The marigold oleoresin used usually contains 10-15% of complete xanthophylls. However, oleoresins with significantly higher complete xanthophyll content, obtained by a process of concentration or purification, can be used. In any event, the soap dosage is adjusted depending on the total quantity of yellow xanthophylls expressed as Lutein.

The water-dispersible form consists of marigold soap, which is prepared according to the usual methods by the process of partial or total saponification of the marigold oleoresin by the action of an alkaline reagent, such as alkaline hydroxides or alkaline earth or metallic alkoxides, in an aqueous or hydroalcoholic means. The best results have been achieved using aqueous potassium hydroxide as a saponification reagent in an aqueous mean.

With regard to the quantity and characteristics of the added marigold soap, as shown previously, there is a wide range of possibilities. In fact, small additions of soap create a significant improvement in pigmenting power as well as a considerable stabilizing effect on the Canthaxanthine.

The amount of soap which is added to prepare the formulation is fixed depending on the proportion of total xanthophylls in the marigold oleoresin, expressed as an equivalent quantity in Lutein, against the amount of canthaxanthine used and depending on the colour desired.

Depending on the amounts used of each one of the xanthophylls and auxiliary agents, the prepared formulation will contain between 1 part of total carotenoids per 1000 parts of solution and 500 parts of total carotenoids per each 1000 parts of solution.

Another important aspect related to the specific characteristics of the claimed formulation is based on the nature and amount of excipients and auxiliary agents.

As stated previously, the formulation is prepared from water-dispersible Canthaxanthine which ensures the presence of the agents used in the final product, such as carbohydrates, proteins and, if appropriate, inorganic excipients, which contribute to the increase of pigmenting activity through the additional xanthophyll stability.

Moreover, the presence of soaps, rubbers, waxes and other agents which accompany the saponified marigold extract, which constitutes the other ingredient of the claimed formulation, are fundamental in increasing the stability of all the xanthophylls and consequently its pigmenting efficiency.

The amounts of these auxiliary agents are in proportion to the related amounts of canthaxanthine and marigold soap used in the preparation of the formulation described, and therefore covers a wide interval of concentrations.

An important factor in the preparation of a good product is agitation. Regardless of the previous type of canthaxanthine formulation, the best results are obtained when it is applied on the marigold soap with strong agitation assuring a good mixture of the ingredients. Once the addition is completed, agitation is continued for a period of between 1 minute and several hours, preferably between 1 and 3 hours, until a perfectly homogenous mixture is achieved. The incorporation of an additional agitation system is useful, Ultra-Turrax or similar, especially when the order of the addition of the canthaxanthine and marigold soap is changed.

In some cases, and with the aim of fluidizing the soap to obtain a more homogenous mixture, the addition of small amounts of an inorganic acid is recommended, such as phosphoric acid, hydrochloric acid, sulphuric acid or similar, or even an organic acid. In these cases, the addition must be done slowly and carefully, and with strong agitation avoiding area of high concentration of acid. In any event, the final pH value must remain above 9.

The temperature, although not a critical parameter, is a considerable influence to the extent that it can fluidify the dispersion means and thereby achieve a more homogenous mixture. Temperature values between 5°C and 95°C are usual. However, the best results have been achieved at temperatures between 25°C and 70°C, adjusting the value depending on the fluidity and therefore the composition of the marigold soap used.

In usual operating conditions, the presence of air does not presume a significant risk. However, it is recommended that an Argon or Nitrogen inert atmosphere be used to obtain optimal results.

A particularly useful characteristic of the formulation described is that it is a liquid. Therefore the incorporation of this formulate can be done in different ways among which spraying or nebulising over the feed is considered the most appropriate from a practical point of view, the feed preferably already granulated thus decreasing the thermal shock which the granulating process would cause on the xanthophylls.

A perfectly homogenous distribution of xanthophylls is thus ensured across all the feed and a fine protective film is also developed by the presence of the auxiliary agents within the formulation which prevents thermal or oxidative degradation.

This aspect is one of the reasons that justifies a greater pigmentation efficiency and a greater homogeneity in colour development, both on the skin and in the egg yolk, compared to the use of powdered xanthophylls separately, as is usually used.

Next, a series of examples are described of how to carry out the invention described, which do not have any limiting character.

### Example 1.-

The saponification of the marigold oleoresin is carried out in accordance with the following proportions of raw materials:
190g marigold oleoresin
76g 50% potassium hydroxide
and heated to 70-80°C with strong agitation until saponification is completed.

It is acidified with phosphoric acid and water added to adjust the concentration up to an approximate value of between 20 and 25% soap.

The mixture prepared this way is poured with strong agitation onto a preparation obtained from canthaxanthine, sucrose and gelatine, together with auxiliary agents, in conditions such that the final proportion of total marigold xanthophylls compared to canthaxanthine is 15:1.

Agitation is maintained for one hour, letting it gradually reach room temperature.

The result is a perfectly homogenous dispersion of xanthophylls in the aqueous means, usable as a pigmenting additive in animal feed.

### Example 2.-

The saponification of the marigold oleoresin is carried out, obtaining the corresponding soap in accordance with example 1, using
400g marigold oleoresin
160g 50% potassium hydroxide
and heating to 80°C with strong agitation until the saponification is complete.

The equivalent amount of canthaxanthine in the form of beadlets is added to this soap, in conditions such that the final proportion of total marigold xanthophylls compared to canthaxanthine is 10:1.

Water is added and acidified using phosphoric acid and maintaining agitation for 30 minutes.

An ultraturrax agitator is connected and agitation is maintained for 2 hours while it cools to room temperature.

A homogenous preparation of xanthophylls in the aqueous medium is obtained which is suitable as an additive in animal feed.

### Example 3.-

Saponification of the marigold oleoresin is carried out in accordance with example 2, maintaining the heat for 45 minutes, without completing, therefore, the saponification.

In these conditions, maintaining the temperature around 60-65°C, the equivalent amount of canthaxanthine in the form of beadlets is added, in conditions such that the final proportion of total marigold xanthophylls compared to canthaxanthine is 20:1.

Heat is maintained until the saponification of the marigold oleoresin esters is complete and, without cooling, water is added and it is acidified with phosphoric acid, maintaining agitation for 30 minutes.

An ultraturrax agitator is connected and agitates for 30 minutes while letting it cool to room temperature.

The result is an aqueous dispersion of red and yellow xanthophylls, suitable for use as a pigment in animal feed.

## Claims

1. Water-dispersible formulation of carotenoids in liquid form, comprising a mixture of Canthaxanthine and saponified marigold oleoresin, which contains between 1 and 500 parts of total carotenoids for each 1000 parts of solution suitable for use as a pigmenting agent in the animal feed industry.

2. Formulation of carotenoids according to claim 1 in which the marigold oleoresin used has been subjected to a process of partial saponification of the xanthophyll esters.

3. Formulation of carotenoids according to claim 1 in which the marigold oleoresin used has been subjected to a process of total saponification of the xanthophyll esters.

4. Procedure for the preparation of the formulation described in claim 1 which consists of the following phases:
- Obtaining of saponified marigold oleoresin by partial or total saponification of the marigold oleoresin;
- Preparation of the water-dispersible Canthaxanthine formulation;
- Mixing of both products in controlled agitation and temperature conditions.

5. Procedure according to claim 4 in which the marigold oleoresin is subjected to a partial or total saponification of its xanthophyll esters, through the action of alkaline reagents, such as alkaline hydroxides or alkaline earth or metal alkoxides, in an aqueous medium.

6. Procedure according to claim 4 in which the dispersible product containing the Canthaxanthine is prepared in microencapsulated, dispersion or gel form.

7. Procedure according to claim 4 in which the saponified marigold oleoresin and water-dispersible canthaxanthine mixture is carried out with strong agitation until a homogenous mixture is achieved.

8. Procedure according to claims 4 to 7 in which the agitation of the mixture is maintained for between 1 and 3 hours, depending on the agitation conditions used, until a totally homogenous mixture is achieved.

9. Procedure according to claims 4 to 8 in which the temperature of the mixture is kept between 5°C and 95°C, preferably between 25°C and 70°C, depending on the composition and viscosity of the ingredients used and on the agitation conditions, with the aim of maintaining sufficient fluidity to achieve a homogenous mixture.

10. Procedure according to claim 4 in which the mixture is performed in the absence of air by the use of an inert atmosphere of Argon or Nitrogen.

11. Use of the formulation obtained according to claim 1 in the preparation of additives, premixtures or products for animal feed.

12. Use of the formulation obtained according to claim 1 in the preparation of drinks for animals.

13. Use of the formulation obtained according to claim 1 for the pigmentation of eggs and animal tissue.

14. Method of applying the liquid formulation of claim 1 to animal feed by spraying or nebulising.

## Patentansprüche

1. Wasserdispergierbare Carotinoidzusammensetzung in flüssiger Form, umfassend eine Mischung aus Canthaxanthin und verseiftem Tagetes-Oleoresin, welche zwischen 1 und 500 Teilen an Gesamt-Carotinoiden pro 1000 Teile einer zur Verwendung als Pigmentierungsmittel in der Tierfuttermittelindustrie geeigneten Lösung enthält.

2. Carotinoidzusammensetzung nach Anspruch 1, in welcher das verwendete Tagetes-Oleoresin einem teilweisen Verseifungsprozess der Xanthophyllester unterzogen worden ist.

3. Carotinoidzusammensetzung nach Anspruch 1, in welcher das verwendete Tagetes-Oleoresin einem vollständigen Verseifungsprozess der Xanthophyllester unterzogen worden ist.

4. Verfahren zur Herstellung der in Anspruch 1 beschriebenen Zusammensetzung, welches aus den folgenden Phasen besteht:
- Erhalten von verseiftem Tagetes-Oleoresin durch teilweises oder vollständiges Verseifen des Tagetes-Oleoresins;
- Herstellen der wasserdispergierbaren Canthaxanthinzusammensetzung;
- Mischen der beiden Produkte unter kontrollierten Rühr- und Temperaturbedingungen.

5. Verfahren nach Anspruch 4, wobei das Tagetes-Oleoresin in einem wässrigen Medium durch Einwirkung von Alkali-Reagenzien, wie Alkali-Hydroxiden oder Erdalkali- oder Metallalkoxiden, einer teilweisen oder vollständigen Verseifung seiner Xanthophyllester unterzogen wird.

6. Verfahren nach Anspruch 4, wobei das das Canthaxanthin enthaltende dispergierbare Produkt in Form von Mikrokapseln, Dispersion oder Gel hergestellt wird.

7. Verfahren nach Anspruch 4, wobei die Mischung des verseiften Tagetes-Oleoresins und des wasserdispergierbaren Canthaxanthins unter starkem Rühren erfolgt, bis eine homogene Mischung erreicht ist.

8. Verfahren nach Ansprüchen 4 bis 7, wobei, abhängig von den verwendeten Rührbedingungen, das Rühren der Mischung für zwischen 1 und 3 Stunden beibehalten wird, bis eine vollkommen homogene Mischung erreicht ist.

9. Verfahren nach Ansprüchen 4 bis 8, wobei, abhängig von der Zusammensetzung und Viskosität der verwendeten Bestandteile und den Rührbedingungen, die Temperatur der Mischung zwischen 5 °C und 95 °C gehalten wird, bevorzugt zwischen 25 °C und 70 °C, mit dem Ziel der Aufrechterhaltung einer ausreichenden Fließfähigkeit, um eine homogene Mischung zu erreichen.

10. Verfahren nach Anspruch 4, wobei die Mischung in Abwesenheit von Luft unter Verwendung einer inerten Atmosphäre aus Argon oder Stickstoff erfolgt.

11. Verwendung der gemäß Anspruch 1 erhaltenen Zusammensetzung für die Herstellung von Zusatzstoffen, Vormischungen oder Produkten für Tierfuttermittel.

12. Verwendung der gemäß Anspruch 1 erhaltenen Zusammensetzung für die Herstellung von Getränken für Tiere.

13. Verwendung der gemäß Anspruch 1 erhaltenen Zusammensetzung für die Pigmentierung von Eiern und Tiergewebe.

14. Verfahren zum Auftragen der flüssigen Formulierung nach Anspruch 1 auf Tierfutter mittels Sprühen oder Zerstäuben.

## Revendications

1. Formulation de caroténoïdes sous forme liquide pouvant être dispersée dans l'eau, comprenant un mélange de canthaxanthine et d'oléorésine de soucis saponifiée, qui contient un total de 1 à 500 parts de caroténoïdes pour chaque 1000 parts de solution appropriée pour être utilisée comme agent de pigmentation dans l'industrie de l'alimentation animale.

2. Formulation de caroténoïdes selon la revendication 1 dans laquelle l'oléorésine de soucis utilisée a été soumise à un procédé de saponification partielle des esters de xanthophylle.

3. Formulation de caroténoïdes selon la revendication 1 dans laquelle l'oléorésine de soucis utilisée a été soumise à un procédé de saponification totale des esters de xanthophylle.

4. Procédure pour la préparation de la formulation décrite dans la revendication 1 qui se compose des phases suivantes :
- l'obtention d'oléorésine de soucis saponifiée par la saponification partielle ou totale de l'oléorésine de soucis ;
- la préparation de la formulation de canthaxanthine pouvant être dispersée dans l'eau ;
- le mélange des deux produits dans des conditions d'agitation et de température régulées.

5. Procédure selon la revendication 4 dans laquelle l'oléorésine de soucis est soumise à une saponification partielle ou totale de ses esters de xanthophylle, par l'action de réactifs alcalins, tels que des hydroxydes alcalins ou des alcoxydes métalliques ou terreux alcalins, dans un milieu aqueux.

6. Procédure selon la revendication 4 dans laquelle le produit pouvant être dispersé contenant la canthaxanthine est préparé sous forme de gel ou de dispersion ou sous forme micro-encapsulée.

7. Procédure selon la revendication 4 dans laquelle le mélange d'oléorésine de soucis saponifiée et de canthaxanthine pouvant être dispersée dans l'eau est réalisé avec une forte agitation jusqu'à obtention d'un mélange homogène.

8. Procédure selon les revendications 4 à 7 dans laquelle l'agitation du mélange est maintenue pendant 1 à 3 heures, en fonction des conditions d'agitation utilisées, jusqu'à obtention d'un mélange totalement homogène.

9. Procédure selon les revendications 4 à 8 dans laquelle la température du mélange est maintenue entre 5 °C et 95 °C, de préférence entre 25 °C et 70 °C, en fonction de la composition et de la viscosité des ingrédients utilisés et des conditions d'agitation, dans le but de maintenir une fluidité suffisante afin d'obtenir un mélange homogène.

10. Procédure selon la revendication 4 dans laquelle le mélange est effectué en l'absence d'air par l'utilisation d'une atmosphère inerte d'argon ou d'azote.

11. Utilisation de la formulation obtenue selon la revendication 1 pour la préparation d'additifs, de pré-mélanges ou de produits destinés à l'alimentation animale.

12. Utilisation de la formulation obtenue selon la revendication 1 pour la préparation de boissons pour animaux.

13. Utilisation de la formulation obtenue selon la revendication 1 pour la pigmentation d'oeufs et de tissus animaux.

14. Procédé d'application de la formulation liquide selon la revendication 1 à l'alimentation animale par pulvérisation ou par nébulisation.
